# EUROPÄISCHE PATENTANMELDUNG

(11) **EP 1 090 927 A1**
(43) Veröffentlichungstag der Anmeldung: **11.04.2001**
(21) Anmeldenummer: 99120119.5
(22) Anmeldetag: 08.10.1999
(51) Int. Cl.: C07K 16/30, A61K 39/395, A61K 51/10, G01N 33/563, C12N 15/13, C12N 15/63, C12N 1/21

(54) **Polypeptide (scFv) zur Detektion und Elimination CA19-9 antigen positiver Zellen**

(71) Anmelder: Abken, Hinrich, 56414 Meudt-Dahlen (DE)
(72) Erfinder: Abken, Hinrich, 56414 Meudt-Dahlen (DE)
(74) Vertreter: Helbing, Jörg, Dr.Dipl.-Chem.

(57) **Zusammenfassung**

Die Erfindung betrifft Polypeptide, die Bindungsspezifität für das CA19-9 Antigen aufweisen, deren Herstellung und Verwendung zur Detektion und Elimination CA19-9 Antigen positiver Zellen, d. h. zur Tumordiagnostik und -therapie. Die Erfindung betrifft weiterhin radiomarkierte Derivate dieser Polypeptide, deren Herstellung sowie deren Verwendung in Radioimmunoassays, Radioscintigraphie und Radioimmunotherapie sowie Kopplungsprodukte dieser Polypeptide mit Indikatorsubstanzen oder pharmakologisch wirksamen Substanzen.

SEQUENZPROTOKOLL

<110> Abken, Hinrich

<120> Polypeptide zur Detektion und Elimination CA19-9
      Antigen positiver Zellen

<130> 991789ep

<140>
<141>

<160> 3

<170> PatentIn Ver. 2.1


<210> 1
<211> 741
<212> DNA
<213> Künstliche Sequenz

<220>
<221> CDS
<222> (14)..(733)

<220>
<223> Beschreibung der künstlichen Sequenz:DNA die für
      scFv des Antikörpers 1116-NS-19-9 kodiert

<400> 1
ggcccagccg gcc atg gcc cag gtc aag ctg cag cag tca gga gga ggc     49
               Met Ala Gln Val Lys Leu Gln Gln Ser Gly Gly Gly
                 1               5                  10

ttg gtg caa cct gga gga tcc atg aaa ctc tct tgt gct gcc tct gga     97
Leu Val Gln Pro Gly Gly Ser Met Lys Leu Ser Cys Ala Ala Ser Gly
        15                  20                  25

ttc act ttt agt gac gcc tgg atg gac tgg gtc cgc cag tct cca gag    145
Phe Thr Phe Ser Asp Ala Trp Met Asp Trp Val Arg Gln Ser Pro Glu
    30                  35                  40

aag ggg ctt gag tgg gtt gct gaa att gga aac aaa ggt aat aat cat    193
Lys Gly Leu Glu Trp Val Ala Glu Ile Gly Asn Lys Gly Asn Asn His
 45                  50                  55                  60

gca acg tac tat gct gag tct gtg aaa ggg agg ttc acc gtc tca aga    241
Ala Thr Tyr Tyr Ala Glu Ser Val Lys Gly Arg Phe Thr Val Ser Arg
                 65                  70                  75

gat gat tcc aaa agt aga gtc tac ctg caa atg aac agc tta aga gtt 289
Asp Asp Ser Lys Ser Arg Val Tyr Leu Gln Met Asn Ser Leu Arg Val
            80                  85                  90

gaa gac act ggc act tat tac tgt acc acg cgg ttt gct tac tgg ggc 337
Glu Asp Thr Gly Thr Tyr Tyr Cys Thr Thr Arg Phe Ala Tyr Trp Gly
        95                  100                 105

caa ggg acc acg gtc acc gtc tcc tca ggt gga ggc ggt cca ggc gga 385
Gln Gly Thr Thr Val Thr Val Ser Ser Gly Gly Gly Gly Pro Gly Gly
    110                 115                 120

ggt ggc tct ggc ggt ggc gga tcg gac atc cag atg acg cag tct cca 433
Gly Gly Ser Gly Gly Gly Gly Ser Asp Ile Gln Met Thr Gln Ser Pro
125                 130                 135                 140

tct tcc atg tat gca tct cta gga gag aga gtc act atc act tgc aag 481
Ser Ser Met Tyr Ala Ser Leu Gly Glu Arg Val Thr Ile Thr Cys Lys
                145                 150                 155

gcg agt cag gac att aat agc tat tta agc tgg ttc cag cag aaa cca 529
Ala Ser Gln Asp Ile Asn Ser Tyr Leu Ser Trp Phe Gln Gln Lys Pro
            160                 165                 170

ggg aaa tct cct aag acc ctg atc tac cgt gca aac aga ttg gta gat 577
Gly Lys Ser Pro Lys Thr Leu Ile Tyr Arg Ala Asn Arg Leu Val Asp
        175                 180                 185

ggg gtc cca tca agg ttc agt ggc ggt gga tct ggg caa gat tat tct 625
Gly Val Pro Ser Arg Phe Ser Gly Gly Gly Ser Gly Gln Asp Tyr Ser
    190                 195                 200

ctc acc atc agc agc ctg gag tat gaa gat atg gga att tat tat tgt 673
Leu Thr Ile Ser Ser Leu Glu Tyr Glu Asp Met Gly Ile Tyr Tyr Cys
205                 210                 215                 220

cta cag tat gat gag ttt cct cgg acg ttc ggt gga ggg acc aag ctg 721
Leu Gln Tyr Asp Glu Phe Pro Arg Thr Phe Gly Gly Gly Thr Lys Leu
                225                 230                 235

gaa ata aaa cgg gcggccgc 741
Glu Ile Lys Arg
            240

<210> 2
<211> 240
<212> PRT
<213> Künstliche Sequenz

<223> Beschreibung der künstlichen Sequenz:DNA die für
      scFv des Antikörpers 1116-NS-19-9 kodiert

<400> 2
Met Ala Gln Val Lys Leu Gln Gln Ser Gly Gly Gly Leu Val Gln Pro
  1               5                  10                  15

Gly Gly Ser Met Lys Leu Ser Cys Ala Ala Ser Gly Phe Thr Phe Ser
             20                  25                  30

Asp Ala Trp Met Asp Trp Val Arg Gln Ser Pro Glu Lys Gly Leu Glu
         35                  40                  45

Trp Val Ala Glu Ile Gly Asn Lys Gly Asn Asn His Ala Thr Tyr Tyr
     50                  55                  60

Ala Glu Ser Val Lys Gly Arg Phe Thr Val Ser Arg Asp Asp Ser Lys
 65                  70                  75                  80

Ser Arg Val Tyr Leu Gln Met Asn Ser Leu Arg Val Glu Asp Thr Gly
                 85                  90                  95

Thr Tyr Tyr Cys Thr Thr Arg Phe Ala Tyr Trp Gly Gln Gly Thr Thr
            100                 105                 110

Val Thr Val Ser Ser Gly Gly Gly Gly Pro Gly Gly Gly Gly Ser Gly
        115                 120                 125

Gly Gly Gly Ser Asp Ile Gln Met Thr Gln Ser Pro Ser Ser Met Tyr
    130                 135                 140

Ala Ser Leu Gly Glu Arg Val Thr Ile Thr Cys Lys Ala Ser Gln Asp
145                 150                 155                 160

Ile Asn Ser Tyr Leu Ser Trp Phe Gln Gln Lys Pro Gly Lys Ser Pro
                165                 170                 175

Lys Thr Leu Ile Tyr Arg Ala Asn Arg Leu Val Asp Gly Val Pro Ser
            180                 185                 190

Arg Phe Ser Gly Gly Gly Ser Gly Gln Asp Tyr Ser Leu Thr Ile Ser
        195                 200                 205

Ser Leu Glu Tyr Glu Asp Met Gly Ile Tyr Tyr Cys Leu Gln Tyr Asp
    210                 215                 220

Glu Phe Pro Arg Thr Phe Gly Gly Gly Thr Lys Leu Glu Ile Lys Arg
225                 230                 235                 240

<210> 3
<211> 31
<212> PRT
<213> Künstliche Sequenz

<220>
<223> Beschreibung der künstlichen Sequenz:Linkerpeptid

<400> 3
Gly Thr Thr Val Thr Val Ser Ser Gly Gly Gly Gly Pro Gly Gly Gly
1               5                   10                  15

Gly Ser Gly Gly Gly Gly Ser Asp Ile Gln Met Thr Gln Ser Pro
            20                  25                  30

## Beschreibung

Die Erfindung betrifft Polypeptide, die Bindungsspezifität für das CA19-9 Antigen aufweisen, deren Herstellung und Verwendung zur Detektion und Elimination CA19-9 Antigen positiver Zellen, d. h. zur Tumordiagnostik und -therapie. Die Erfindung betrifft weiterhin radiomarkierte Derivate dieser Polypeptide, deren Herstellung sowie deren Verwendung in Radioimmunoassays, Radioscintigraphie und Radioimmunotherapie sowie Kopplungsprodukte dieser Polypeptide mit Indikatorsubstanzen oder pharmakologisch wirksamen Substanzen.

Magen- und Pankreas-Carcinome haben eine Inzidenz von 24.000 bzw. 28.000 in USA mit einer jährlichen Todesrate beim Pankreas-Carcinom von annähernd 28.000, beim Magen-Carcinom von 14.000. Für Europa gelten ähnliche Zahlen. Die mittlere Überlebenszeit nach Diagnosestellung eines Pankreas-Carcinoms liegt bei ca. 6 Monaten. Diese infauste Prognose ist insbesondere dadurch bedingt, daß sich die meisten Pankreas-Tumore bei Diagnosestellung in einem nicht mehr operablen Stadium befinden, so daß ein therapeutischer Ansatz ohne Einfluß auf den Krankheitsverlauf bleibt. Diese Situation verdeutlicht zwei gravierende Mängel, die eine erfolgreiche Therapie in der überwiegenden Mehrzahl verhindern: (1) effektive Verfahren, den Tumor *in vivo* frühzeitig aufspüren, und (2) Verfahren, bei fortgeschrittenem Tumor-Stadien eine ausreichende Tumorregression zu erzeugen, die ein operatives Vorgehen ermöglicht.

Anstrengungen zur Verbesserung dieser Situation werden durch das Fehlen eines Reagenz erschwert, das in bildgebenden Verfahren *in vivo* zum Nachweis und zur Lokalisation von Primärtumor und Metastasen eingesetzt werden kann. Ein therapeutischer Ansatz wird weiterhin dadruch erschwert, daß ein Reagenz zum spezifischen *in vivo* drug-targeting des Pankreas- und Magen-Carcinoms nicht zur Verfügung steht.

Das CA19-9 Antigen ist ein Monosialogangliosid, das in der immunhistochemischen Analyse mit Hilfe des Antikörpers 1116-NS-19-9 (Maus IgG1) bei zahlreichen Magen- und Pankreas-Carcinomen detektiert wird (Koprowski et al., Somatic Cell Genet. 5, 957-971, 1979; Koprowski et al., U.S. Pat. 4,471,057; Hybridomklon ATCC HB-8059).

Obwohl der Antikörper seit über 20 Jahren bekannnt und verfügbar ist und in der *in vitro* Diagnostik erfolgreich verwendet wird, sind Versuche, den Antikörper 1116-NS-19-9 für die *in vivo* Diagnostik einsetzbar zu machen, bisher mit begrenztem Erfolg geblieben. Es zeigte sich, daß die Gewebspenetration des Antikörpers bei soliden Tumoren zu gering ist, um Tumore zuverlässig *in vivo* zu detektieren (Kazumoto et al., Ann. Nucl. Med. 7, 39 - 44, 1993). Ein Einsatz des Antikörpers konnte deswegen bisher nicht empfohlen werden (Allum, Baillieres Clin. Gastroenterol. 4, 853 - 867, 1990). Um die Gewebemarkierung durch den Antikörper zu verbessern, wurde vorgeschlagen, cobra-venom-factor (CVF) Konjugate des CA19-9 Antikörpers zur Vorbehandlung einzusetzen (Juhl et al., Cancer Res. 55(23suppl), 5749s-5755s, 1995). Jedoch konnte lediglich eine zweifache Verbesserung der Tumormarkierung durch den Antikörper erzielt werden. Die Verwendung eines anti-CA19-9 F(ab')2 Antikörpers führte nur in 1 von 7 Patienten zur zuverlässigen Detektion des primären Tumors *in vivo* (Naruki et al., Ann. Nucl. Med. 8, 163-169, 1994).

Die mehrfache Applikation des anti-CA19-9 Antikörpers für *in vivo* Zwecke ist begrenzt. Da es sich bei dem Antikörper 1116-NS-19-9 (ATCC HB-8059) um einen Maus-Antikörper handelt, sind "HAMA" Immunreaktionen ("human anti-mouse antibodies") bei wiederholten Applikationen zu erwarten. Weitere Versuche, den Antikörper durch Modifikationen für einen diagnostischen oder therapeutischen in-vivo-Einsatz verwendbar zu machen, sind bisher erfolglos geblieben.

Die Aufgabe der Erfindung bestand darin, Reagentien zum Nachweis (*in vivo* targeting) des Magen- und Pankreas-Carcinoms zur Verfügung zu stellen.

Überraschenderweise wurde gefunden, daß ein spezielles Fragment eines Antikörpers gegen das CA19-9 Antigen, nämlich ein rekombinantes, antikörperähnliches Molekül, das die variable Region der schweren Kette (VH) und der leichten Kette (VL) eines anti-CA19-9 Antikörpers umfaßt (d. h. ein sogenanntes "single chain fragment of variable regions", scFv), mehrere überraschend vorteilhafte Eigenschaften vereint, die zusammen einen *in vivo* Einsatz ermöglichen.

Gegenstand der Erfindung ist somit
(1) ein Polypeptid, umfassend die Oligopeptide
   (A) bestehend aus der variablen Region der schweren Kette (VH) eines anti-CA19-9-Antikörpers oder eines Fragments desselben mit Bindungsspezifität für das CA19-9 Antigen und
   (B) bestehend aus der variablen Region der leichten Kette (VL) eines anti-CA19-9-Antikörpers oder eines Fragments desselben mit Bindungsspezifität für das CA19-9 Antigen,
   wobei die Oligopeptide (A) und (B) direkt oder durch ein Brückenpeptid (C) miteinander verknüpft sind;
(2) eine DNA, kodierend für das in (1) definierte Polypeptid;
(3) ein Vektor, enthaltend die in (2) definierte DNA;
(4) ein Wirtsorganismus der mit dem in (3) definierten Vektor transformiert ist;
(5) ein Verfahren zur Herstellung des in (1) definierten Polypeptids, umfassend das Kultivieren des transformierten Wirtsorganismus, wie in (4) definiert;
(6) die Verwendung des in (1) definierten Polypeptids zur Herstellung von Arzneimittel und Diagnostika, die zum Nachweis, zur Lokalisierung und/oder zur Eliminierung von CA19-9 Antigen tragenden Zellen, insbesondere Tumorzellen geeignet sind;
(7) ein Radiomarkiertes Polypeptid, wobei das Polypeptid wie in (1) definiert ist;
(8) ein Verfahren zur Herstellung des in (7) definierten radiomarkierten Polypeptids, umfassend das Umsetzen des in (1) definierten Polypeptids mit dem Radioisotop;
(9) die Verwendung des in (7) definierten radiomarkierten Polypeptids zur Herstellung von Arzneimittel und Diagnostika, insbesondere für die Herstellung von Arzneimitteln für die Tumortherapie und für die Herstellung von Mitteln, die für bildgebenden diagnostischen *in vitro* und *in vivo* Verfahren geeignet sind; und
(10) Arzneimittel oder Diagnostika, umfassend das in (1) definierte Polypeptid und/oder das in (7) definierte radiomarkierte Polypeptid.

### Figurenbeschreibung

Fig. 1 zeigt die DNA- und die Aminosäure-Sequenz (frame: one-letter-code) des CA19-9-bindenden Polypeptids. Die VH- und VL-Regionen und das Brückenpeptid sind gekennzeichnet. Das integrierte Peptid (DNA bp 341-433) ist ein bevorzugtes Brückenpeptid und kann durch andere Brückenpeptide ersetzt werden.

Fig. 2: Bindung des Phagenantikörpers SEQ ID NO:2-scFv an indirekt immobilisiertes CA19-9 Antigen aus Tumorzellen
Zur indirekten Immobilisierung des CA19-9 Antigens wurde eine Mikrotiterplatte mit dem monoklonalen Antikörper NS19-9 (2 µg/ml) beschichtet und anschließend mit Lysaten der CA19-9-positiven Tumorzellinie SW948 (■) oder der CA19-9-negativen Tumorzellinie H716 (□) inkubiert. Verdünnungen des SEQ ID NO:2-scFv-M13-Phagenantikörpers wurden auf die Antigen-beschichtete Platte überführt und gebundene Phagenantikörper mit Hilfe eines Peroxidase-gekoppelten anti-M13 Antikörpers und Indikatorfarbreaktion photometrisch bei O.D. 405 nm nachgewiesen.

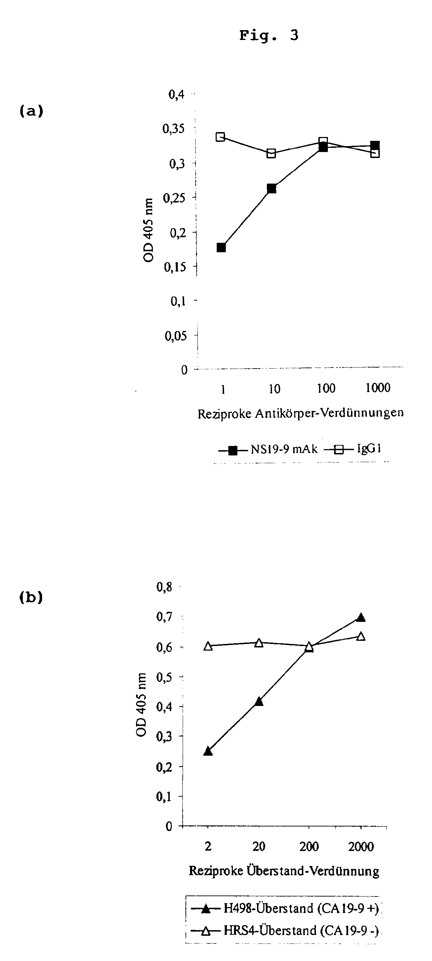

Fig. 3a: Kompetition der Bindung des Phagenantikörpers SEQ ID NO:2-scFv-M13 an indirekt immobilisiertes CA19-9 Antigen durch den monoklonalen Antikörper NS19-9
Zur indirekten Immobilisierung des CA19-9 Antigens wurde eine Mikrotiterplatte mit anti-CA19-9 Antikörper NS19-9 beschichtet (2 µg/ml NS19-9 mAk) und anschließend mit 100 µl Überstand der CA19-9 sezernierenden Tumorzellen H498 inkubiert. Die Inkubation des SEQ ID NO:2-scFv-M13 Phagenantikörpers erfolgte in Gegenwart verschiedener Verdünnungen des monoklonalen anti-CA19-9 Antikörpers NS19-9 (■) oder eines Antikörpers gleichen Isotyps (IgG) mit irrelevanter Spezifität (□) (max. Konzentration 10 µg/ml). Die photometrische Detektion (O.D. 405 nm) des gebundenen Phagenantikörpers SEQ ID NO:2-scFv-M13 erfolgte mit Hilfe eines Peroxidase-gekoppelten anti-M13 Antikörpers und Farbindikatorreaktion.

Fig. 3b: Kompetition der Bindung des Phagenantikörpers SEQ ID NO:2-scFv-M13 an indirekt immobilisiertes CA19-9 Antigen durch lösliches CA19-9 Antigen CA19-9 Antigen aus dem Kulturüberstand der CA19-9 positiven Tumorlinie H489 wurde auf NS19-9 Antikörper beschichteten Mikrotiterplatten immobilisiert (2 µg/ml NS19-9 Antikörper, 100 µl H498-Überstand). Die Inkubation des SEQ ID NO:2-scFv-M13 Antikörpers erfolgte in Gegenwart von Verdünnungen des CA19-9-haltigen Kulturüberstandes der CA19-9-positiven Tumorzellinie H498 (▲) oder der CA19-9-negativen Tumorzellinie HRS4 (Δ). Die photometrische Detektion (O.D. 405 nm) des gebundenen Phagenantikörpers SEQ ID NO:2-scFv-M13 erfolgt mit Hilfe eines Peroxidase gekoppelten anti-M13 Antikörpers und Farbindikatorreaktion.

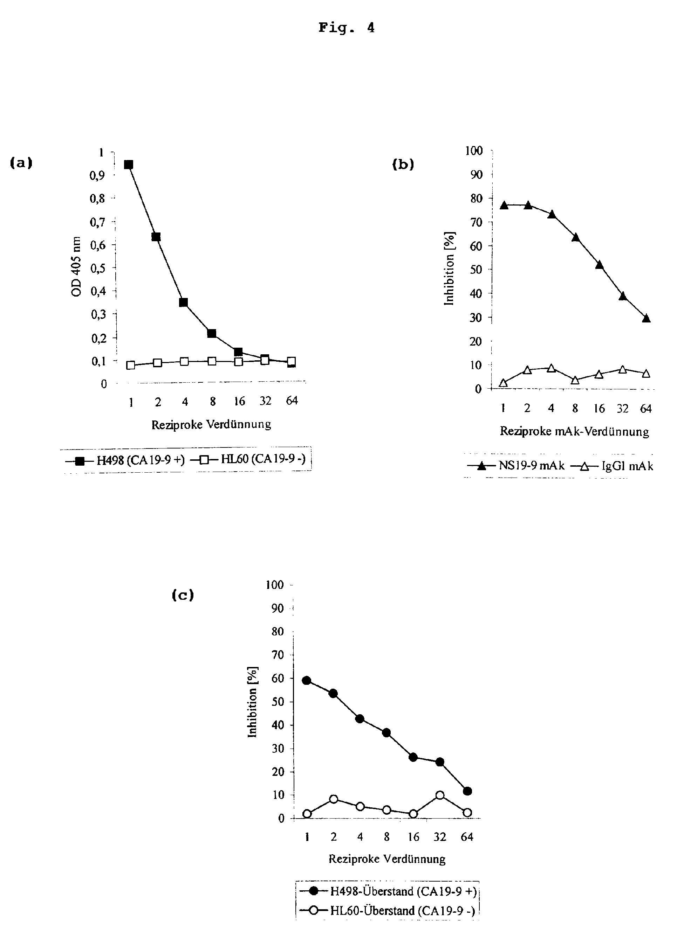

Fig. 4a: Bindung des Fusionsproteins SEQ ID NO:2-scFv-CH2CH3 an immobilisiertes CA19-9 Antigen
Zur indirekten Immobilisierung des CA19-9 Antigens wurde eine Mikrotiterplatte mit dem monoklonalen Antikörper NS19-9 (2 µg/ml) beschichtet und anschließend mit CA19-9 Antigen-haltigem Kulturmedium der CA19-9 positiven Tumorzellinie H498 (■) oder mit dem Kulturüberstand der CA19-9 negativen Tumorlinie HL60 (□) inkubiert. Das Fusionsprotein SEQ ID NO:2-scFv-CH2CH3 wurde in Verdünnungen auf die Antigen-beschichtete Platte gegeben und das gebundene Fusionsprotein anschließend mit Hilfe eines Biotin-gekoppelten anti-human IgG(Fc) Antikörpers, der gegen die CH2CH3 Domäne des Fusionsproteins gerichtet ist, und einer Streptavidin-gekoppelten Peroxidase und Farbindikatorreaktion photometrisch detektiert (O.D. 405 nm). Die Daten sind Mittelwerte aus zwei unabhängigen Versuchsansätzen.

Fig. 4b: Kompetition der Bindung des Fusionsproteins SEQ ID NO:2-scFv-CH2CH3 an immobilisiertes CA19-9 Antigen durch den monoklonalen Antikörper NS19-9 Zur Immobilisierung des CA19-9 Antigens wurde eine Mikrotiterplatte mit dem anti-CA19-9 monoklonalen Antikörper NS19-9 beschichtet (2 µg/ml NS19-9 Antikörper) und anschließend mit CA19-9 Antigen aus H498 Zellkulturüberstand inkubiert. Die Inkubation des SEQ ID NO:2-scFv-CH2CH3 Fusionsproteins erfolgte in Gegenwart von Verdünnungen des monoklonalen Antikörpers NS19-9 (■) oder eines Kontrol-Antikörpers gleichen Isotyps (IgG1) mit irrelevanter Spezifität (□). Die photometrische Detektion (O.D. 405 nm) des gebundenen Fusionsproteins SEQ ID NO:2-scFv-CH2CH3 erfolgte mit Hilfe eines Biotin-gekoppelten anti-human IgG(Fc) Antikörpers und einer Streptavidin-gekoppelten Peroxidase. Die Daten sind Mittelwerte aus zwei unabhängigen Versuchsansätzen.

Fig. 4c: Kompetition der Bindung des Fusionsproteins SEQ ID NO:2-scFv-CH2CH3 an immobilisiertes CA19-9 Antigen durch lösliches CA19-9 Antigen Zur Immobilisierung des CA19-9 Antigens wurde eine Mikrotiterplatte mit dem anti-CA19-9 Antikörper NS19-9 beschichtet (2 µg/ml NS19-9 Antikörper) und anschließend mit CA19-9 Antigen aus H498 Zellkulturüberstand inkubiert. Auf dem indirekt immobilisierten CA19-9 Antigen wurde das Fusionsprotein SEQ ID NO:2-scFv-CH2CH3 in Gegenwart von Verdünnungen des Kulturmediums der CA19-9 positiven Tumorzellinie H498 (●), die große Mengen CA19-9 Antigen sezerniert, oder des Kulturüberstandes der CA19-9-negativen Tumorlinie HL60 (o) inkubiert. Die photometrische Detektion (O.D. 405 nm) des gebundenen Fusionsproteins SEQ ID NO:2-scFv-CH2CH3 erfolgte mit Hilfe eines Biotin-gekoppelten anti-human IgG(Fc) Antikörpers und einer Streptavidin-gekoppelten Peroxidase. Die Ergebnisse sind Mittelwerte aus zwei unabhängigen Versuchsansätzen.

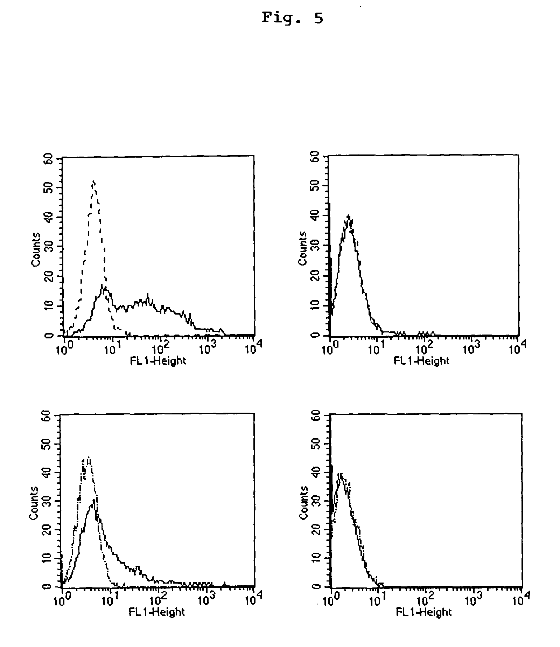

Fig. 5: FACS-Analyse der Bindung des Fusionsproteins SEQ ID NO:2-CH2CH3 auf CA19-9-positiven Tumorzellen
Zellen der CA19-9-positiven Tumorzellinie LoVo5176 sowie der CA19-9-negativen Tumorzellinie H716 wurden mit dem monoklonalen Antikörper NS19-9 (A) und dem Fusionsprotein SEQ ID NO:2-CH2CH3 (B) inkubiert (durchgezogene Linien). Als Kontrollen diente der monoklonale Antikörper HRS3 (mit Spezifität für das CD30 Antigen) bzw. das Fusionsprotein HRS3-scFv-CH2CH3 (gepunktete Linien). Die Detektion gebundener Fusionsproteine erfolgte mit einem FITC-gekoppelten anti-murin IgG(Fc) Antikörper (A) bzw. einem anti-human IgG(Fc) Antikörper (B) und der Durchflußzytometrie (FACS).

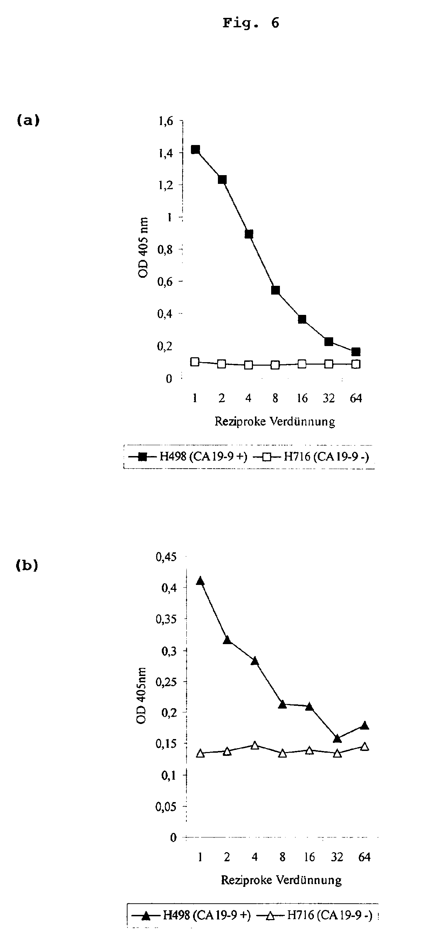

Fig. 6a, b: Bindung des Fusionsproteins SEQ ID NO:2-CH2CH3-IL2 an immobilisiertes CA19-9 Antigen
Zur Immobilisierung des CA19-9 Antigens wurde eine Mikrotiterplatte mit dem monoklonalen Antikörper NS19-9 (2 µg/ml) beschichtet und anschließend mit CA19-9 Antigen-haltigem Kulturüberstand der CA19-9 positiven Tumorzellinie H498 (■,▲) oder mit dem Kulturüberstand der CA19-9-negativen Tumorlinie H716 (□, Δ) inkubiert. Das Fusionsprotein SEQ ID NO:2-CH2CH3-IL2 wurde in den angegebenen Verdünnungen auf immobilisiertem CA19-9 Antigen inkubiert. Gebundenes Fusionsprotein wurde (a) mit Hilfe eines Biotin-gekoppelten anti-human IgG(Fc) Antikörpers, der die CH2CH3 Domäne des Fusionsproteins bindet, oder (b) mit Hilfe eines Biotin-gekoppelten anti-human IL2 Antikörpers und anschließender Farbindikatorreaktion photometrisch detektiert (O.D. 405 nm).

Das vorstehend unter (1) definierte Polypeptid wird nachfolgend näher beschrieben. Gemäß der vorliegenden Erfindung stammen die beiden Oligopeptide vorzugsweise von gleichen Antikörpern. Andererseits ist es bevorzugt, daß eines oder beide Oligopeptide von dem Antikörper 1116-NS-19-9 stammen. Die Hybridomzellen, die diese Antikörper produzieren, können von der American Type Culture Collection, Rockville; MD, unter der Bezugsnummer ATCC HB-8059 erworben werden kann.

Bei der Verknüpfung der Oligopeptide (A) und (B) kann sowohl das Oligopeptid (A) als auch das Oligopeptid (B) das N-terminale Bestandteil des erfindungsgemäßen Polypeptids sein. In einer bevorzugten Ausführungsform sind die Oligopeptide (A) und (B) über ein Brückenpeptid (C) (nachfolgend auch "Linker" oder Linkerpeptid" genannt) miteinander verknüpft.

Als Brückenpeptid (C) können alle Peptide eingesetzt werden, die eine Faltung und Anordnung der Oligopeptide (A) und (B) zueinander erlauben, daß eine effiziente Antigenbindung gewährleistet ist. Bevorzugte Brückenpeptide bestehen aus einem kurzem, flexiblen Oligopeptid mit 5 bis 50, insbesondere mit 20 bis 50 Aminosäureresten. Besonders bevorzugt sind Ser und Gly (SG)-reiche Brückenpeptide, die z. B. bis 10 Aminosäurereste lagern. Diese SG-reichen Brückenpeptide sind vorzugsweise modular aufgebaut und umfassen SG-reiche Elemente, z. T. durch Pro getrennt. Die SG-reichen Elemente sind zwischen 2 und 20 Aminosäuren lang. Es reicht dabei ein SG-Element aus, um die o. g. Funktionen zu erfüllen. Ein Prolinrest in der Mitte des Brückenpeptids dient der Erzeugung eines Winkels in der Polypeptidkette. An der Stelle des Prolins können auch andere Aminosäuren wie z. B. Gly, Ser, Asp und Asn verwendet werden.

Diese Brückenpeptide weisen vorzugsweise die folgende Struktur

-A-(GlyₘSerₙ)ₓ-B-C-D-(GlyₒSerₚ)_{y}-E

und insbesondere die Struktur

-(GlyₘSerₙ)ₓ-Pro-(GlyₒSerₚ)_{y}-

auf, wobei
A, B, D, E unabhängig voneinander eine Bindung, ein Aminosäurerest oder ein Polypeptid mit bis zu 5 Aminosäureresten sind,
C ausgewählt ist aus Pro, Gly, Ser, Asp und Asn und insbesondere Pro ist,
n, m, o und p unabhängig voneinander ganze Zahlen von 0 bis 10 sind und
x und y unabhängig voneinander ganze Zahlen von 1 bis 5 sind.

Ein besonders bevorzugtes Brückenpeptid (C) ist in SEQ ID NO:3 gezeigt.

In einer besonders bevorzugten Ausführungsform der Erfindung weist das erfindungsgemäße Polypeptid (1) die Aminosequenz des SEQ ID NO:2 auf, das aus VH und VL des 1116-NS-19-9 Antikörpers in der Anordnung VH-Linkerpeptid-VL besteht.

Zur Generierung eines scFv Moleküls stehen dem Fachmann mehrere Verfahren zur Verfügung, deren Gemeinsamkeit darin besteht, ausgehend von einem Antikörper produzierenden Hybridom oder einer geeigneten rekombinanten Bibliothek, die cDNA kodierend für die variable Region der schweren Immunglobulin-Kette (VH) und die cDNA für die der leichten Kette (VL) so zu rekombinieren, daß die rekombinante DNA für eine scFv Polypetid-Kette in der Anordnung VH-linker-VL oder VL-linker-VH kodiert. Diese DNA kann dann in einen geeigneten Wirtsorganismus/Zellsystem exprimiert werden. Geeignete Systeme im Sinne der vorliegenden Erfindung sind bakterielle Expressionssysteme, z. B. E. coli HB2151 unter Verwendung des Vektors pCANBAB 5E oder eurkaryonte Expressionssysteme , z. B. 293T Zellen unter Verwendung des Vektors pRSV (mit Ig kappa-Leader-Peptid).

Es zeigte sich überraschenderweise, daß das erfindungsgemäße Polypeptid, hier exemplarisch für das scFv Molekül SEQ ID NO:2 gezeigt, die Spezifität des parentalen Antikörpers 1116-NS-19-9 für das CA19-9 Antigen bewahrt hat. Das Molekül SEQ ID NO:2 bindet spezifisch an CA19-9 Antigen (Beispiel 1), an CA19-9 Antigentragende Zellen (Beispiel 2, Fig. 5), jedoch nicht an andere getestete Karbohydrat-Antigene und CA19-9 Antigen-negative Zellen (Beispiel 1).

Weiterhin zeigte sich überraschenderweise, daß die Spezifität des anti-CA19-9 scFv auch dann erhalten bleibt, wenn eine weitere Polypeptidkette an das SEQ ID NO:2 Polypeptid gekoppelt wurde. Exemplarisch ist die Kopplung der humanen IgG1 Immunglobulin CH2CH3 Domänen an die scFv Domäne in Beispiel 2 gezeigt.

Entgegen der häufigen Beobachtung, daß Moleküle der Konstruktion VH-linker-VL oder VL-linker-VH in wässrigen Lösungen schlecht löslich sind und zur Präzipitation neigen, zeigt das Molekül SEQ ID NO:2 eine sehr gute Löslichkeit. Weiterhin zeichnet sich das Molekül SEQ ID NO:2 nach Denaturierung durch eine sehr effiziente Renaturierung in eine bindungsaktive Konfiguration aus (Beispiel 4).

Weiterhin zeigt das Molekül SEQ ID NO:2 eine unerwartet gute Gewebspenetration und Akkumulation an CA19-9 positive Zellen, bevorzugt Tumorzellen *in vivo.* Dieses zeigt sich beispielsweise nach intravenöser Applikation des markierten Moleküls SEQ ID NO:1 in Mäuse, die experimentell erzeugte CA19-9 positive Tumore tragen (Beispiel 6). Eine Akkumulation an gesundem Gewebe erfolgt jedoch nicht. Bevorzugte Verwendung des erfindungsgemäßen Moleküls ist die Detektion und Lokalisierung von CA19-9 positiven Zellen *in vivo,* insbesondere Tumore, beispielsweise Magen- und Pankreas-Carcinom, in der bildgebenden Diagnostik.

Weiterhin zeigte sich, daß das Molekül SEQ ID NO:2 nach Bindung an die Antigentragende Zelle nicht internalisiert wird. Diese Eigenschaft ist um so überraschender, da in der überwiegenden Mehrzahl der Rezeptor-Ligand Systeme die gebundenen Liganden internalisiert werden. Dieses gilt um so mehr für Antikörper, die an ein Molekül der äußeren Zellmembran binden. Die fehlende Internalisierung des Moleküls SEQ ID NO:2 gewährleistet außerdem eine lange Verweildauer auf der Zelloberfläche ohne Degradation durch zytoplasmatische Proteasen. Diese Eigenschaft erweist sich besonders vorteilhaft in der Verwendung des SEQ ID NO:2 Moleküls für das drug targeting, pro-drug activation, radioaktive *in-vivo*-Markierung und anderen Verfahren, in denen das Molekül zum targeting verwendet wird.

Das Molekül SEQ ID NO:2 ist frei von konstanten Anteilen der Maus-Immunglobuline, so daß keine ausgeprägte HAMA-Immunreaktion bei wiederholten Applikationen des Moleküls zu erwarten ist. Diese Eigenschaft ermöglicht, im Gegensatz zu den bisher vorhandenen anti-CA19-9 Antikörpern, die Verwendung des SEQ ID NO:2 Moleküls bei *in-vivo*-Verfahren in der Diagnostik und Therapie.

Nach Kopplung des Moleküls SEQ ID NO:2 mit den humanen CH2CH3 Immunglobulin-Domänen zeigte sich weiterhin, daß nach Bindung an CA19-9 positive Tumorzellen eine spezifische Tumor-Lyse durch natürliche Killer-Zellen (NK-Zellen) und Complement vermittelt wird. Gesundes Gewebe oder Tumore, die dieses Antigen nicht tragen, werden nicht lysiert. Eine bevorzugte Verwendung des Moleküls SEQ ID NO:2 nach Kopplung mit Ig CH2CH3, CH2 oder CH3 Domänen ist die Elimination von CA19-9 positiven Tumorzellen *in vivo* und *in vitro.*

Bevorzugter Gegenstand der Erfindung ist die kovalente oder nicht-kovalente Kopplung des anti-CA19-9 scFv Moleküls an eine weitere Vielzahl von Molekülen entsprechend der gewünschten Verwendung. Folgende Modifikationen des erfindungsgemäßen Polypeptids und ihre Verwendungen sollen hier nur beispielhaft genannt werden.

1. Markierung des Polypeptids und insbesondere des Moleküls SEQ ID NO:2 mit Radioisotopen, z.B. 125J, 131J, 99^{m}Tc, 188Re, 186Re, 32P, für die Verwendung *in vitro* und *in vivo,* z.B. im Radioimmunassay oder in der Radioimmunscintigraphie und Radioimmuntherapie. Etablierte Verfahren zur radioaktiven Markierung des Moleküls umfassen die Einführung von Metall-Chelat Gruppen, Peptidsequenzen, die eine radioaktive Markierung erleichtern, C- oder N-terminales Cystein, oder Metallothionin (Dean et al., Technetium and Rhenium in Chemistry and Nuclear Medicine, Raven Press, New York, pp 605-608,1990; Liberatore et al., Europ. J. Nucl. Med. 22: 1326-1329, 1995; Ram u. Buchsbbaum, Cancer 73(suppl3), 769-773, 1994; Zhao et al., Biocnjug. Chem. 10, 424-430, 1999). Beispielhaft sei weiterhin genannt die Kopplung des Moleküls mit Calmodulin und die Beladung mit Calmodulin-bindenden, radioaktiv markierten Molekülen zum Einsatz in der Mehrschritt-Immunscintigraphie (VanEldik und Lukas, Methods Enzymol. 139, 393 - 4055, 1987; Török und Trentham, Biochemistry 33, 12807 - 12820, 1994). Jedoch erfordern zahlreiche Markierungsreaktionen stark reduzierende Reaktionsbedingungen, z.B. bei Markierung mit 186Re, was häufig zur Denaturierung des Antikörpers führt und eine Verwendung des Antikörpers in der Immunscintigraphie oder Radioimmuntherapie unmöglich macht.

Die unerwartet hohe Stabilität der erfindungsgemäßen Moleküle, insbesondere SEQ ID NO:2, gegenüber denaturierenden Reaktionsbedingungen sowie die ungewöhnlich gute Renaturierung nach Denaturierung ermöglicht den Einsatz von Markierungsreaktionen ohne Verlust der Bindungsspezifität. Zur Reinigung bindungsaktiver renaturierter Molekül erweist sich eine Affinitätschromatographie vorteilhaft durch Bindung an das CA19-9 Antigen oder, bei einem Fusionsmolekül bestehend aus SEQ ID NO:2 und einer Detektionsdomäne, beispielhaft der humanen IgG1 CH2CH3 Domäne, durch Bindung an Domänen-spezifische Antikörper.

Nach *in vivo* Applikation akkumuliert das erfindungsgemäße Polypeptid, so z. B. das Molekül SEQ ID NO:2 am Ort des CA19-9 positiven Tumors und seiner Metastasen. Bei Verwendung eines radioaktiv markierten Moleküls können mit Hilfe scintigraphischer Verfahren die Tumore *in vivo* lokalisiert werden. Bei der *in vivo* Detektion im Sinne der vorliegenden Erfindung wird das z. B. mit einem Radioisotop markierte Polypeptid dem Patienten verabreicht und die Bindung an das CA19-9 tragende Gewebe von außerhalb des Körpers detektiert. Auch können andere indirekte oder direkte Verfahren zur Immunscintigraphie eingesetzt werden für den Nachweis von CA19-9 positiven Tumoren *in vivo,* wobei die erfindungsgemäßen Moleküle, bevorzugt SEQ ID NO:2, die spezifische Bindung an den Tumorzellen vermittelt.

Eine weitere Verwendung des erfindungsgemäßen Polypeptids ist die strahlentherapeutische Elimination von CA19-9 positiven Zellen nach Applikation in vivo. Dabei sind direkte oder indirekte radioaktive Markierungen möglich. Beispielhaft sei die direkte Markierung des Moleküls SEQ ID NO:2 mit 188Re oder die indirekte Markierung durch sequentielle oder simultane Applikation eines tag-tragenden Fusionsmoleküls SEQ ID NO:2 -tag und eines radioaktiv markierten anti-tag Moleküls genannt.

Unter "tag" wird in diesem Zusammenhang eine Proteindomäne (kurze Peptidsequenz) verstanden, die den Nachweis des Proteins z. B. mit Hilfe von Antikörpern und/oder die Reinigung des Proteins z. B. mit Hilfe von Ni-Bindung erleichtert. Bevorzugte therapeutische Verwendung des 188Re-markierten Moleküls SEQ ID NO:2 ist die Erzielung einer Tumorreduktion in vivo zur Elimination von Metastasen oder zur Schaffung der Vorraussetzungen für eine erfolgreiche operative Therapie. Durch eine bevorzugte Akkumulation des 188Re-markierten Moleküls SEQ ID NO:2 am Tumor wird eine Akkumulation der Strahlung am Tumor bei gleichzeitiger Minderung der hämatopoietischen Toxizität erzielt, während bei der Verwendung anderer Trägermoleküle oder der Verwendung von externen Strahlenquellen ist häufig eine ausgeprägte Toxizität für Zellen des blutbildenden Systems zu finden (Iznaga-Escobar, Nucl. Med. Biol. 25, 441-447, 1998).

2. Eine weitere Verwendung der erfindungsgemäßen Polypeptide, bevorzugt des Moleküls SEQ ID NO:2, liegt in der Herstellung von modifizierten Molekülen mit "Designer" Effektor Funktionen. Beispielsweise können nach bekannten Verfahren (Übersicht bei Neri et al., Engineering recombinant antibodies for immunotherapy, Cell. Biophys. 27:47-61,1995) verschiedene chimäre, stöchiometrisch definierte Fusions-Moleküle hergestellt werden, beispielhaft durch Konjugationen an andere Polypeptide, an Zuckermoleküle, Lipide oder andere synthetische oder natürliche Substanzen, Chelatoren, oder auch die Kopplung mit pharmakologisch wirksamen Substanzen, z.B. Molekülen mit zytolytischer Aktivität (z.B. Perforin) oder pro-drug Substanzen, Proteine mit Signalfunktionen (z.B. Zytokine) (Beispiel 3), Proteinen mit enzymatischer Aktivität (z.B. Metalloproteinase, Endopeptidasen). Auch ist eine Kopplung mit Nukleinsäuren möglich. Gemeinsam ist diesen Ausführungsformen die Verwendung des erfindungsgemäßen Polypeptids, insbesondere des SEQ ID NO:2, zur Erzielung der Akkumulation einer gewünschten Substanz am Ort der CA19-9 Expression, bevorzugt am Ort des Tumors, *in vivo*.

3. Eine weitere Verwendung ist die Kopplung des erfindungsgemäßen Polypeptids, insbesondere des Moleküls SEQ ID NO:2, an Bindedomänen anderer Moleküle oder Antikörper zur Generierung bi- und multispezifischer Hybridmoleküle, z.B. bispezifische Antikörper. Beispielhaft sei ein rekombinanter anti-CA19-9/anti-CD3 (OKT3) bispezifischer scFv Antikörper genannt zum Einsatz in der Immuntherapie CA19-9 positiver Tumore.

4. In einer weiteren Ausführungsform kann das erfindungsgemäße Polypeptid, insbesondere das Molekül SEQ ID NO:2, mit einer intrazellulären Signalkette fusioniert und in geeigneten Wirtszellen als Signal-transduzierendes Rezeptormolekül exprimiert werden. Überraschend zeigte sich dabei, daß ein durch Bindung des CA19-9 Antigens an SEQ ID NO:2 ein biochemisches Signal auf der fusionierten heterologen Signalpeptidkette ausgelöst wird (Beispiel 5). Dem Fachmann stehen zahlreiche Signalketten-Kombinationen offen, die hier nicht umfassend genannt werden können.

Es konnte gezeigt werden, daß die erfindungsgemäßen Polypeptide zur Lokalisierung und/oder Eliminierung von CA 19-9 Antigen tragenden Zellen geeignet ist. Die Erfindung betrifft somit ebenfalls ein Verfahren zur Aktivierung CA 19-9 tragender Zellen, umfassend die Verabreichung des (gegebenenfalls derivatisierten oder modifizierten) Polypeptids (1) an Mensch oder Tier.

Die Erfindung wird in den folgenden Beispielen näher erläutert.

### Beispiele

### Beispiel 1: Das Einzelkettenmolekül SEQ ID NO:2 hat Bindungsspezifität für das CA19-9 Antigen und bindet an das CA19-9 Antigen aus CA19-9 positiven Tumorzellen.

Die Generierung des Moleküls SEQ ID NO:1 erfolgte mit Hilfe der Phage-Display-Technik (McCafferty et al., Nature 348: 552 - 554 (1990); Winter und Milstein, Nature 349: 293- 299 (1991)), ausgehend von dem Hybridom 1116-NS-19-9 (ATCC HB-8059), der den 1116-NS-19-9 Antikörper mit Spezifität für das CA19-9 Antigen exprimiert (Koprowski et al., Somatic Cell Genet. 5, 957 - 971, 1979).

Aus den Hybridom-Zellen wurde polyA+ mRNS nach bekannten Verfahren gewonnen (Sambrook et al., Molecular Clonig, 2nd Edition, Cold Spring Harbor Laboratory Poress, Cold Spring Harbor, N.Y., pp. 8.11 - 8.13, 1989) und mit Hilfe der reversen Transkriptase und der "primed first-strand reaction mix" Oligonukleotide (Pharmacia Biotech, Freiburg, Cat. No. 27-9400-01) in cDNS umgeschrieben. Die cDNS, kodierend für die variable Region der schweren (V_{H}) und leichten (V_{L}) Immunglobulin Kette, wurde mit Hilfe Immunglobulin-spezifischer Oligonukleotide ("heavy primer 1", "heavy primer 2", "light primer mix", Pharmacia, Cat.Nr. 27-1583-01, 27-1586-01) in einer PCR Reaktion amplifiziert. In einer zweiten PCR-Reaktion wurde die V_{H}- (ca. 340 bp) und V_{L}- cDNS (ca. 325 bp) mit Hilfe einer Verbindungssequenz, kodierend für ein (Gly₄Ser)₃-Peptid (Huston et al., Proc. Natl. Acad. Sci. USA 85: 5879 - 5883, 1988) und der "linker-primer-mix" (Pharmacia, Cat.Nr. 27-1588-01) in einer PCR-Reaktion zu einem Molekül verbunden. Das V_{H}-linker-V_{L} Fragment (scFv) (ca. 750 bp) wurde reamplifiziert, wobei Oligonukleotide ("RS-primer-mix", Pharmacia, Cat.No. 27-1589-01) benutzt wurden, welche die Enden der PCR Produkte mit flankierenden Schnittstellen für die Restriktionsendonukleasen Sfi I (5' V_{H}-Ende) und Not I(3' VL-Ende) ausstatten. Nach Spaltung dieser Restriktionsstellen erlauben die so generierten DNS-Enden eine gerichtete Integration in die Sfi I/Not I linearisierte pCANTAB 5E Phagemid DNS (Pharmacia, Cat.Nr. 29-9401-01). Das scFv-Fragment wird in diesem Phagemid als Fusionsprotein mit dem Phagen Protein gp3 auf der Phagenoberfläche exprimiert.

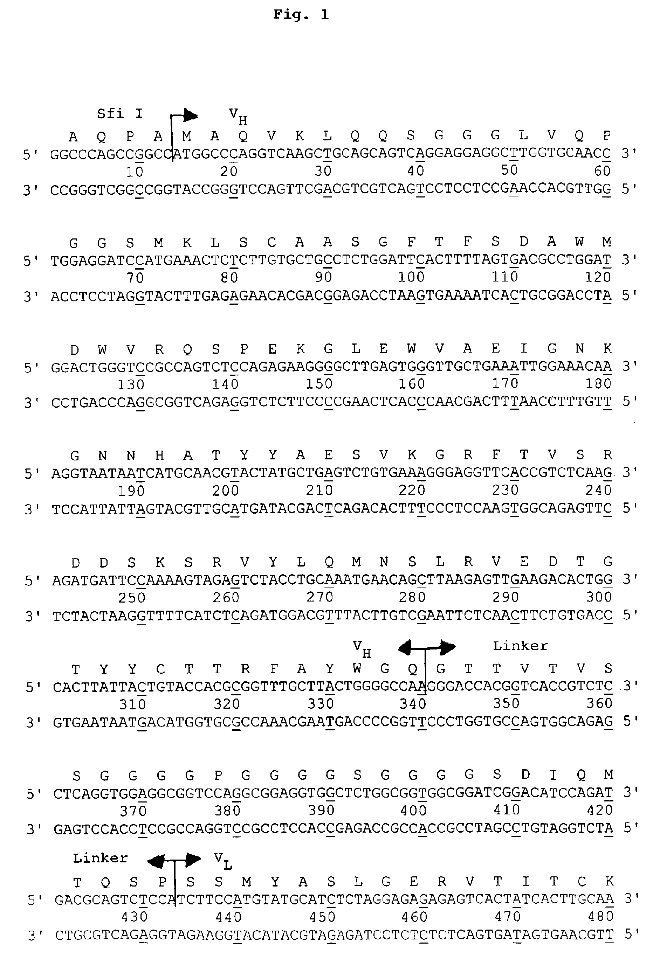

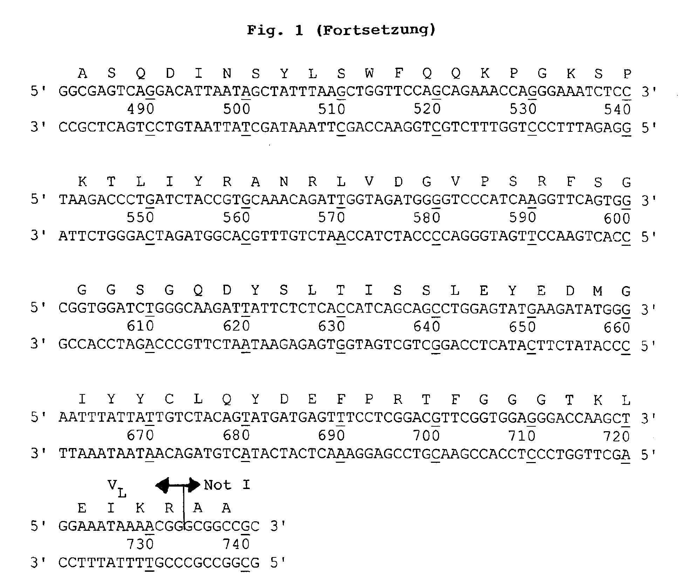

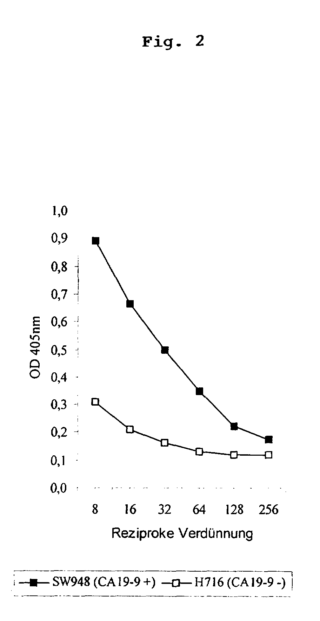

Zur Produktion der M13 Phagen mit dem CA19-9-bindenden scFv Molekül SEQ ID NO:2 wurden TG1 E.coli Bakterien mit der rekombinanten Phagemid-DNS transformiert und anschließend mit M13K07 Helferphagen infiziert. Mit Hilfe der Panning-Technik (Huston et al., Proc. Natl. Acad. Sci. USA 85: 5879 - 5883, 1988) wurden selektiv die Phagen angereichert, die das CA19-9 Antigen zu binden vermögen. Das CA19-9 Antigen bindende, rekombinante scFv Molekül (NS19-9-scFv) hat die in Fig. 1 und SEQ ID NO:1 gezeigte DNS- und die in SEQ ID NO:2 gezeigte Aminosäure-sequenz. Der Phagen-Antikörper SEQ ID NO:2-scFv-M13 wird auf der Oberfläche der Phagen als Fusionsprotein scFv-gp3 exprimiert und bindet spezifisch das CA19-9 Antigen aus CA19-9 positiven Tumorzellen (Fig. 2). Das gp3 Protein des M13 Phagen hat keine Bindungsspezifität.

Die Bindung des SEQ ID NO:2-scFv Fragments ist spezifisch für das CA19-9 Antigen, da die Bindung des SEQ ID NO:2-scFv an CA19-9 Antigen durch den monoklonalen Antikörper NS19-9 (Fig. 3a) und durch lösliches CA19-9 Antigen (Fig. 3b) kompetitiert wird.

### Beispiel 2: Das Polypeptid SEQ ID NO:2 bewahrt auch nach Fusion an die humane Immunglobulin IgG1 CH₂CH₃-Domäne die Bundungsspezifität für das CA19-9 Antigen.

Das CA19-9 bindende scFv-Molekül SEQ ID NO:2 wurde mit einer zweiten, unabhängigen Proteindomäne unter Beibehaltung der Bindungsspezifität für das CA19-9 Antigen zu einem Fusionsprotein verbunden. Dazu wurde die DNA SEQ ID NO:1, die für das anti-CA19-9 scFv, mit der cDNA kodiert, die für die humane IgG1 CH₂CH₃-Domäne kodiert, ligiert. Die resultierende cDNA, die für das scFv- CH₂CH₃-Fusionsprotein kodiert, wird in den pRSV Expressionsvektor inseriert und nach Transfektion in Ag8.653 Zellen exprimiert. Der pRSV Expressionsvektor (Eshhar et al., Proc. Natl. Acad. Sci. USA 90: 720 - 724 (1993)) ist gekennzeichnet durch eine RSV-LTR gesteuerte Expressionskassette mit einem Immunoglobulin kappa-Leichtketten-Leader-Peptid. Dieses gewährleistet, daß das exprimierte Fusionsprotein in den Kulturüberstand sezerniert wird. Das Fusionsprotein SEQ ID NO:2-scFv-CH₂CH₃ wird aus dem serumfreien Kulturüberstand transfizierter Ag8.653-Zellen mit Hilfe der Affinitätschromatographie durch Bindung an anti-human Fc-Antikörper gekoppelte Agarose isoliert. Die SEQ ID NO:2-scFv-Domäne hat im Fusionsmolekül die Bindungsspezifität für das CA19-9 Antigen bewahrt. Die konservierte Bindungsspezifität wurde durch Bindung des SEQ ID NO:2-scFv- CH2CH3-Fusionsproteins an CA19-9 Antigen (Fig. 4a) und durch Kompetition der Bindung durch den monoklonalen Antikörper NS19-9 (Fig. 4b) und lösliches CA19-9 Antigen (Fig. 4c) nachgewiesen. Das Fusionsmolekül SEQ ID NO:2-scFv CH₂CH₃ bindet spezifisch an CA19-9 positive Tumorzellen, nicht jedoch an CA19-9 negative Tumorzellen (Fig. 5). Diese Daten zeigen, daß die CA19-9 bindende Domäne SEQ ID NO:2 einersetis die gleiche Bindungsspezifität für das CA19-9 Antigen hat wie der monoklonale NS19-9 Antikörper, andererseits auch dann die Bindungsspezifität beibehält, wenn die SEQ ID NO:2-scFv-Domäne Teil eines Fusionsproteins mit anderen funktionellen Domänen ist.

### Beispiel 3: Die Fusion der Moleküls SEQ ID NO:2-CH₂CH₃ an humanes Interleukin-2 (IL-2) generiert ein bifunktionales Protein mit Bindungsspezifität für das CA19-9 Antigen.

Durch Fusion des Moleküls SEQ ID NO:2-CH₂CH₃ (aus Beispiel 2) mit Interleukin-2 (IL-2) wurde ein bifunktionales Fusionsprotein generiert, das Bindungsspezifität für das CA19-9 Antigen ausprägt und zugleich das IL-2 Protein trägt. Hierzu wurde die DNA.SEQ ID NO:1-CH₂CH₃ (aus Beispiel 2) mit der cDNA, die für humanes IL-2 kodiert, fusioniert und in die Expressionskassette des Expressionsvektors pRSV inseriert. Der pRSV Expressionsvektor (Eshhar et al., Proc. Natl. Acad. Sci USA 90: 720 - 714 (1993)) ist gekennzeichnet durch eine RSV-LTR gesteuerte Expressionskassette mit einem Immunoglobulin kappa-Leichtketten-Leader-Peptid, so daß das exprimierte Fusionsprotein in den Kulturüberstand sezerniert wird. Nach Transfektion der Expressionskassette in Ag8.653 Zellen wurde das Fusionsprotein SEQ ID NO:2-CH₂CH₃-IL-2 aus dem Kulturübersand durch Affinitätschromatographie durch Bindung an einem anti-human-Fc-Antikörper, der die CH₂CH₃-Domäne bindet, gereinigt.

Das Fusionsprotein SEQ ID NO:2-CH₂CH₃-IL-2 ist gekennzeichnet durch ein pharmakologisch wirksames Zytokin (IL-2), fusioniert mit der CA19-9 bindenden Domäne SEQ ID NO:2, wobei die Bindungsspezifität des scFv für das CA19-9 Antigen auch in dieser Konfiguration des Fusionsproteins erhalten geblieben ist. Die Bindung des Fusionsproteins SEQ ID NO:2-CH₂CH₃-IL-2 an CA19-9 ist in Fig. 6a, b dokumentiert.

### Beispiel 4: Das Molekül SEQ ID NO:2 bewahrt nach Denaturierung und Renaturierung die Bindungsspezifität für das CA19-9 Antigen.

Das lösliche Molekül SEQ ID NO:2 (ohne Phagenanteil) wird nach Transfektion der SEQ ID NO:1 DNA in pCANTAB 5E Vektor in E. coli HB2151 Bakterien und Induktion mit IPTG produziert und akkumuliert im Periplasma der Bakterien. Die Isolierung des SEQ ID NO:2-Proteins aus dem Periplasma der Bakterien erfolgt durch Inkubation der Bakterien in 1/5 x (v/v) TES Puffer (1 x TES: 200 mM Tris-HCI, 0,5 mM EDTA, 500 mM Sucrose, pH 8,0) für 30 min bei 4 °C und Ammoniumsulfatfällung des Bakterienüberstandes. Das Präzipitat wird in PBS renaturiert. Das renaturierte SEQ ID NO:2-Molekül wurde hinsichtlich der Spezifität der Bindung an CA19-9 Antigen mit dem nativen SEQ ID NO:2-gp3 Phagenmolekül verglichen. Hierbei zeigte sich, daß das SEQ ID NO:2-Molekül nach Denaturierung und Renaturierung in der löslichen Form dieselbe Bindungsspezifität zu dem CA19-9 Antigen aufweist wie der SEQ ID NO:2-gp3 Phagenkörper, dessen Isolierung keine De- und Renaturierung erfordert.

### Beispiel 5: Das Molekül SEQ ID NO:2 als Domäne vermag durch Antigenbindung die fusionierte Signaldomäne zu aktivieren.

Es wurde ein rekombinantes Transmembranmolekül generiert, dessen extrazelluläre Domäne durch das CA19-9 bindenden Molekül SEQ ID NO:2 und dessen Transmembran- und intrazelluläre Domäne durch die γ-Kette des FcεRI Rezeptors gebildet wird. Dieses Fusionsmolekül soll dienen zur spezifischen zellulären Aktivierung von immunokompetenten Zellen (z. B. T-Zellen) nach Kontakt mit CA19-9 Antigen trangenden Zellen (z. B. Tumorzellen).

Die DNA, kodierend für SEQ ID NO:2-CH₂CH₃ (aus Beispiel 2) wurde in die Snabl und BamHI Stelle des Vektors pRSV-γ (Eshhar et al. Proc. Natl. Acad. Sci. USA 90: 720 - 724 (1993)), inseriert unter Deletion der SP6 scFv-Domäne des Vektors. Die neu rekombinierte Vektor-DNA wurde in Maus-MD45-T-Zellen mit Hilfe der Elektroporation transfiziert und stabile Zellklone isoliert, die den gewünschten SEQ ID NO:2-CH₂CH₃-γ-Rezeptor auf der Oberfläche exprimieren.

Rezeptor tragende MD45 Zellen wurden hinsichtlich ihrer Eigenschaft, nach Kontakt mit CA19-9 positiven Tumorzellen spezifisch aktiviert zu werden, geprüft. Dazu wurden rezeptortragende MD45-Zellen und als Kontrolle nicht-transfizierte MD45-Zellen (je 10⁵ Zellen) in parallelen Ansätzen mit CA19-9+ H498 Tumorzellen und mit CA19-9-H716-Tumorzellen (je 10⁵ Zellen) kokultiviert. Nach 48 Stunden wurde die Menge sezerniertes IL-2 im Kulturüberstand als Maß für die zelluläre Aktivierung bestimmt. Es zeigte sich, daß SEQ ID NO:2-CH₂CH₃-γ-Rezeptor tragende MD45-Zellen durch Kokultur mit CA19-9-positiven H498 Tumorzellen aktiviert werden, nicht jedoch durch CA19-9-negative H716-Tumorzellen. Nicht-transfizierte MD45-Zellen werden weder durch H498 noch durch H716-Zellen aktiviert. Weiterhin zeigt dieses Ergebnis, daß die CA19-9 bindende extrazelluläre Domäne SEQ ID NO:2 nach Bindung des zellgebundenen CA19-9 Antigens ein Signal an die fusionierte intrazelluläre γ-Signalkette weiterleitet, die ihrerseits die zelluläre Aktivierung einleitet.

### Beispiel 6: Das Molekül SEQ ID NO:2 akkumuliert in vivo an Zellen mit CA19-9 Expression.

Das lösliche Protein SEQ UD NO:2 (aus Beispiel 1) wurde mit ¹²⁵J unter Verwendung des Bolton-Hunter-Reagenz radioaktiv markiert und von ungebundenem ¹²⁵J gereinigt. Die Bindungsspezifiät des ¹²⁵J-SEQ ID NO:2-Moleküls *in vitro* ist gleich der des nichtmarkierten SEQ ID NO:2-Moleküls. Zur Erzeugung von Tumoren wurden CA19-9 positive H498-Zellen und als Kontrolle CA19-9-H716-Zellen subkutan in Nacktmäuse (nu/nu) injiziert (ca. 10⁶ Zellen pro Injektion und Tier). Nach 5 - 8 Tagen bildete sich jeweils ein subkutaner Tumor von 2 - 4 mm Durchmesser.

Am Tag 7 nach Injektion der Tumorzellen wurde ¹²⁵J-SEQ ID NO:2 intravenös in tumortragende Mäuse appliziert (5 µg Protein/Maus). Nach 24 Stunden wurde die Verteilung der Radioaktivität in der Maus durch Ganzkörper-Autoradiographie bestimmt. Es zeigte sich, daß die Radioaktivität am H498 Zelltumor (CA19-9+) akkumuliert ist, nicht jedoch am H716-Zelltumor (CA19-9-). Die Organe der Mäuse zeigen geringe Radioaktivität in gleicher Verteilung bei H498-Tumor wie bei H716-Tumor tragenden Mäusen.

## Patentansprüche

1. Polypeptid, umfassend die Oligopeptide
(A) bestehend aus der variablen Region der schweren Kette (VH) eines anti-CA19-9-Antikörpers oder eines Fragments desselben mit Bindungsspezifität für das CA19-9 Antigen und
(B) bestehend aus der variablen Region der leichten Kette (VL) eines anti-CA19-9-Antikörpers oder eines Fragments desselben mit Bindungsspezifität für das CA19-9 Antigen,
wobei die Oligopeptide (A) und (B) direkt oder durch ein Brückenpeptid (C) miteinander verknüpft sind.

2. Polypeptid nach Anspruch 1, wobei die Oligopeptide (A) und (B) durch ein Brükkenpeptid (C) miteinander verknüpft sind und das Brückenpeptid (C) vorzugsweise aus 5 bis 50, insbesondere aus 20 bis 50 Aminosäureresten besteht und besonders bevorzugt eine Ser-Gly-reiche Teilsequenz, insbesondere mit 5 bis 10 Aminosäureresten, ist.

3. Polypeptid nach Anspruch 1 oder 2, wobei das Brückenpeptid (C) eine Teilsequenz mit der Struktur
-(GlyₘSerₙ)ₓ-Pro-(GlyₒSerₚ)_{y}-
umfaßt, wobei n, m, o und p unabhängig voneinander ganze Zahlen von 0 bis 10 und x und y unabhängig voneinander ganze Zahlen von 1 bis 5 sind, oder wobei das Brückenpeptid die Aminosäuresequenz von SEQ ID NO:3 aufweist.

4. Polypeptid nach einem oder mehreren der Ansprüche 1 bis 3, wobei die Oligopeptide (A) und (B) vom gleichen Antikörper stammen.

5. Polypeptid nach einem oder mehreren der Ansprüche 1 bis 3, wobei eines oder beide Oligopeptide (A) und (B) von dem Antiköper 1116-NS-19-9 (ATCC HB-8059) stammen.

6. Polypeptid nach Anspruch 1, das die in SEQ ID NO:2 gezeigte Aminosäuresequenz aufweist oder ein Fragment desselben mit Bindungsspezifität für das CA19-9 Antigen.

7. Polypeptid nach einem oder mehreren der Ansprüche 1 bis 6, wobei das Polypeptid mit weiteren funktionellen Substanzen, insbesondere weiteren Peptiden, Zytikonen, Enzymen, Indikatorsubstanzen, pharmakologisch wirksame Substanzen, radioaktiven Isotopen, Toxinen und/oder Farbstoffen verknüpft ist.

8. DNA, die für das in Ansprüchen 1 bis 7 definierte Polypeptid kodiert.

9. Vektor, enthaltend die in Anspruch 8 definierte DNA.

10. Wirtsorganismus, der mit dem in Anspruch 9 definierten Vektor transformiert ist.

11. Verfahren zur Herstellung des in Ansprüchen 1 bis 6 definierten Polypeptids, umfassend das Kultivieren des transformierten Wirtsorganismus gemäß Anspruch 10.

12. Verwendung des Polypeptids nach Ansprüchen 1 bis 7 zur Herstellung von Arzneimitteln und Diagnostika, die zum Nachweis, zur Lokalisierung und/oder zur Eliminierung von CA19-9 Antigen tragenden Zellen, insbesondere Tumorzellen geeignet sind.

13. Radiomarkiertes Polypeptid, wobei das Polypeptid wie in Ansprüchen 1 bis 6 definiert ist.

14. Radiomarkiertes Polypeptid gemäß Anspruch 13, wobei das Radioisotop direkt oder mittels einer Chelatormoleküls an das Polypeptid gebunden ist.

15. Radiomarkiertes Polypeptid gemäß Anspruch 13 oder 14, wobei das Radioisotop ausgewählt ist aus 125J, 131J, 99^{m}Tc, 188Re, 186Re und 32P.

16. Verfahren zur Herstellung des radiomarkierten Polypeptids gemäß Ansprüchen 13 bis 15, umfassend das Umsetzen des Polypeptids gemäß Ansprüchen 1 bis 6 mit dem Radioisotop.

17. Verwendung des radiomarkierten Polypeptids gemäß Ansprüche 13 bis 15 zur Herstellung von Arzneimitteln für die Tumortherapie oder von Diagnostika.

18. Arzneimittel oder Diagnostika, umfassend das Polypeptid gemäß Ansprüchen 1 bis 7 und/oder das radiomarkierte Polypeptid gemäß Anspruch 13 bis 15.
